Europäisches Patentamt

European Patent Office

Office européen des brevets

(11) Publication number: **0 095 346 B1**

(12) **EUROPEAN PATENT SPECIFICATION**

(45) Date of publication of patent specification: **07.04.93**

(21) Application number: **83302898.8**

(22) Date of filing: **20.05.83**

The file contains technical information submitted after the application was filed and not included in this specification

(51) Int. Cl.⁵: **C12N 5/00, C12P 21/08, C12N 15/00, C12R 1/91, A61K 39/02**

(54) Hybrid cell lines producing monoclonal antibodies directed against treponema.

(30) Priority: **26.05.82 US 381929**

(43) Date of publication of application:
**30.11.83 Bulletin 83/48**

(45) Publication of the grant of the patent:
**07.04.93 Bulletin 93/14**

(84) Designated Contracting States:
**BE CH DE FR GB IT LI NL SE**

(56) References cited:

**INFECTION AND IMMUNITY, vol. 35, no. 3, March 1982, pages 974-978; C.SAND PETERSEN et al.: "Purification of a reiter treponemal protein antigen that is immunologically related to an antigen in treponema pallidum".**

**GENETIC ENGINEERING, vol. 2, 1980, pages 31-46; SAU-PING KWAN et al.: "Production of monoclonal antibodies".**

**INFECTION AND IMMUNITY, vol. 36, no. 3, June 1982, pages 1076-1085; STELLA M.ROBERTSON et al.: "Murine monoclonal antibodies specific for virulent Treponema pallidum (Nichols)".**

**Scand. J. Immunol. 13, 143-50 (1981)**

**FEMS Microbiology Letters 14, 61-64 (May 1982)**

(73) Proprietor: **BOARD OF REGENTS THE UNIVERSITY OF TEXAS SYSTEM**
**Office of General Council, 201 West 7th Street**
**Austin, Texas 78701(US)**

(72) Inventor: **Kettman, John R.**
**3119 Barton Road**
**Carrollton Texas 75007(US)**
Inventor: **Norgard, Michael V.**
**4217 Early Morn Drive**
**Plano Texas 75075(US)**

(74) Representative: **Lord, Hilton David et al**
**Marks & Clerk 57-60 Lincoln's Inn Fields**
**London WC2A 3LS (GB)**

EP 0 095 346 B1

**Description**

The present invention relates to the production of monoclonal antibodies; and, in particular, to hybrid cell lines capable of continuously producing monoclonal antibodies directed against Treponema bacterial pathogens.

In recent years, the capability to produce monoclonal antibodies specific for the immunogenic determinants of bacterial cells and toxins has provided a new vista of diagnostic and immunotherapeutic agents.

The bacterial genus Treponema is associated with a variety of pathogenic diseases. Of particular significance, Treponema pallidum is a sexually transmitted bacterial pathogen of man responsibe for syphilis. Very little is known about T. pallidum, despite decades of strenuous efforts to understand this organism. The gross lack of information stems primarily from the fact that T. pallidum is one of the very few bacterial pathogens for man that cannot be grown in vitro like other human pathogens. Consequently, researchers attempting to elucidate the nature of the organism and the disease it produces have been confined to cultivating T. pallidum in the testicles of laboratory rabbits.

Untreated syphilis in man is a severe, chronic, and very complex disease that can often be extremely difficult to diagnose. Limitations with current diagnostic tools and the absence of a vaccine have allowed syphilis to flourish at the estimated frequency of approximately 350,000 cases per year in the United States alone, even with the availability of effective penicillin treatment.

Moreover, there are other treponemal diseases which perhaps have a more worldwide impact with respect to morbidity and mortality. Briefly, the treponemes that cause yaws, pinta, and bejel are treponemes morphologically and serologically indistinguishable from T. pallidum. These diseases are very serious worldwide, especially in the so-called third world countries. These diseases are believed to be transmitted through normal person-to-person contact, as opposed to T. pallidum, which is a sexually transmitted agent. As a result these particular diseases are highly contagious and devastating to large populations thereby eluding effective control.

Many attempts at vaccine development over past decades using whole sells, extracts, or adjuvant preparations of T. pallidum or other non-pathogenic treponemes have failed. One study reported the successful vaccination of rabbits with T. pallidum (Nichols) attenuated by γ-irradiation. There are several major drawbacks to this approach as a potential immunization scheme for humans. These include the impracticality of preparing massive amounts of freshly isolated and freshly irradiated treponemes, as well as the dangers associated with hypersensitivity reactions by the recipient to contaminating rabbit proteins present in treponemal suspensions.

Syphilis and other related pathogenic treponemal disease research continues to lag far behind other areas of bacterial infectious disease. In particular, the complexity of the humoral response to T. pallidum infection and the inability to obtain large amounts of T. pallidum cells for subsequent fractionation of constituent antigens have severely hampered identification of the specific immunogens of T. pallidum that are responsible for eliciting protective immunity in rabbits and man. The specific T. pallidum immunogens that remain unidentified and uncharacterized may hold the key to immunological approaches for the control of syphilis.

The somatic cell fusion of plasmacytoma cell lines with treponemal-sensitized spleen or lymph node cells to produce monoclonal antibodies specific for a treponemal species determinant provides a new and innovative way to circumvent major obstacles of the past. Once successfully carried out, a virtually limitless supply of monospecific antibodies for Treponema pallidum antigens will be readily available at all times. These monospecific antibodies could then be used to analyze the antigenic components of the Treponema immunogens. The isolation and characterization of specific Treponema immunogens through the use of the new hybridoma cell fusion technology may provide the materials and insights needed to understand the pathogenesis and immunological control of syphilis, yaws, pinta, and bejel through vaccine development.

The invention seeks to develop continuous hybrid cell lines which produce monoclonal antibody directed against treponemal antigens. Selected cell lines are capable of continuously producing a set of monospecific antibodies that are identical with respect to combining site specificity to a single antigenic determinant exhibited by a treponemal bacteria.

We have been able to provide continuous hybridoma cell lines which elaborate and secrete highly specific and homogenous monoclonal antibodies to treponemal antigens, in particular Treponema pallidum.

Thus, the present invention provides monoclonal antibody produced by a continuous hybrid cell line, which monoclonal antibody is characterised in that:

(i) it is capable of reacting with sonicated T. pallidum;

(ii) it reacts with that antigenic determinant recognised by monoclonal antibodies produced by hybridoma deposit ATCC HB 8133 or HB 8134.

2

In order to obtain a suitable continuous hybrid cell line, an animal may first be immunised with Treponema bacteria to develop lymphocytes and their differentiated progeny which produce antibodies directed against a priming antigenic determinant. The lymphocytes are recovered and fused with myeloma, plasmacytoma, or hybridoma cells to form somatic cell hybrids. The cell hybrids are cultured, selected, isolated and propagated in tissue culture. Thereafter, the hybrid cell lines are capable of indefinitely producing monoclonal antibodies to the selected immunising antigens.

In accordance with the present invention there are provided continuous hybrid cell lines that produce monoclonal antibody as defined. Continuous cell lines have been isolated which produce monoclonal antibody directed specifically against T. pallidum antigens or against treponemal group antigens (antibody which cross reacts among several of the Treponema species), provided that it is characterised as defined. Moreover, there are provided complement fixing monoclonal antibodies which are capable of immobilising virulent treponemes.

The following discussion is in terms of the preferred embodiments of this invention, which represent the best mode known to the Applicants at the time of this application.

In accordance with the processes of this invention, test animals are stimulated in vitro for antibody production by immunization with a preparation containing Treponema bacteria. Applicants have directed their preferred embodiment to immunizing mice with a T. pallidum inoculum extracted from rabbit testicles.

Alternatively, normal and immune differentiated lymphocytes capable of producing antibody can be isolated from test animals, cultured, and primed with Treponema in vitro to generate cells appropriate for producing lymphocyte hybridomas. For example, such methods of in vitro stimulation with mitogens and/or antigens have been described by Robertson et al, Microbiology 1980 pp. 181-185 (1980) and Kettman et al, J. Immunol. Methods 39:203-222 (1980) or the method splenic fragment culture as described by Press et al, Eur. J. Immunol. 4:155-159 (1974).

The route and schedule of immunizing the host animal or cultured antibody producing cells therefrom are generally in keeping with established and conventional techniques for antibody stimulation and production. Applicants have employed mice as the test model although it is contemplated that any mammalian subject including human subjects or antibody producing cells therefrom can be manipulated according to the processes of this invention to serve as the basis for production of human hybrid cell lines.

After immunization, immune lymphoid cells are fused with myeloma, plasmacytoma, or hybridoma cells (hereinafter referred to collectively as myeloma cells) to generate a hybrid cell line which can be cultivated and subcultivated indefinitely, to produce large quantities of monoclonal antibodies. For purposes of this invention, the immune lymphoid cells selected for fusion are lymphocytes and their normal differentiated progeny, taken either from lymph node tissue or spleen tissue from immunized animals. Applicants prefer to employ immune spleen cells, since they offer a more concentrated and convenient source of antibody producing cells with respect to the mouse system.

The myeloma cells provide the basis for continuous propagation of the fused hybrid. Myeloma cells are tumor cells derived from plasma cells which show preference for bone marrow. Plasmacytoma cells are neoplastic cells derived from plasma cells. In particular, Applicants prefer to use lymphocyte hybridoma cells which secrete no immunoglobulin. Lymphocyte hybridoma cells are cells generated by the fusion of myeloma or plasmacytoma cells with normal differentiated lymphoid cells. Myeloma, plasmacytoma, and hybridomas can be selected to be devoid of immunoglobulin synthesis.

The particular species of animal from which the myeloma and immunized antibody producing cells are derived are not critical, in that it is possible to fuse cells of one species with another. However, it is preferred that the source of immunized antibody producing cells and myeloma be from the same species.

Generally the fusion techniques employed are according to the procedures set out by Kohler et al, Eur. J. Immunol. 6:11-19 (1976) and Kennett et al, Lymphocyte Hybridomas - Current Topics in Microbiology and Immunology 81:77-91 (1978) Springer-Verlag, New York. Fusion is generally accomplished by adding a suspension of antibody producing cells to the myeloma cells in growth medium and thereafter centrifuging to form a pellet.

The fused hybrids are next screened for antibody production specific for treponemal antigens. A hybridoma which secretes antibody specific for, treponemal antigenic determinant is selected and cultured to establish a continuous cell line with stable genetic coding. The cell line can be stored and preserved in any of a number of conventional ways, including freezing and storage under liquid nitrogen. A frozen cell line can be revived and cultured indefinitely with resumed synthesis and secretion of monoclonal antibodies specific for the selected antigenic determinant. The secreted antibody is recovered from tissue culture supernatant by conventional precipitation, ion exchange, affinity chromatography, or the like. The recovered antibody can be freeze dried and stored under refrigeration for at least several weeks without significant loss of activity.

The following examples are offered to illustrate a particular embodiment of the invention but they are not intended to limit it.

A. Preparation of Antigens

T. pallidum (Nichols) was used for sensitizing mice and as a source of antigen in the radioimmunoassay (RIA). The organisms were cultivated in the testicles of New Zealand White rabbits, previously examined upon receipt for the absence of clinical and serological (VDRL non-reactive) evidence of Treponema paraluis-cuniculi infection; the animals were subsequently housed individually at 18-20°C with antibiotic-free food and water given ad libitum. Approximately 12 days following each intratesticular injection of 2 x 10⁷ T. pallidum in one ml of serum-saline extraction medium [0.01M sodium phosphate buffer, pH 7.2, 0.85% (wt/vol) NaCl, 50% (vol/vol) heat-inactivated (56°C, 30 min) normal rabbit serum] per testicle, treponemes were extracted aerobically from minced testicles by rotary shaking at 180 rpm (23°C) for 30 min using 10 ml of extraction medium per testicle. Gross rabbit testicular tissue debris was removed from the treponemal suspension by centrifugation twice at 500 x g for 10 min at 23°C in a 50 ml conical polypropylene centrifuge tube. Treponemes in suspension were then examined for motility, concentrated by centrifugation at 16,000 x g for 20 min, suspended in phosphate buffered saline (PBS) (0.01M sodium phosphate buffer, 0.85% NaCl, pH 7.2), and enumerated by darkfield microscopy. "Fresh" treponemes were used immediately upon isolation to prepare RIA plates, which could be stored at -70°C. "Aged" treponemes were produced by storing suspensions of T. pallidum in PBS at 4°C for 7 days before binding to microliter plate wells for RIA.

T. phagedenis biotype Reiter (Reiter treponeme) was used in a RIA to identify antibodies directed against Treponema group antigens. The organisms were maintained at 23°C and transferred monthly in BBL Thioglycollate Medium 135C (without indicator) containing 10% (vol/vol) heat-inactivated (56°C, 30 min.) sterile bovine serum. Cells for use in RIA were cultivated for 5 days at 35°C in large screw capped tubes containing 40 ml of BBL Spirolate Broth supplemented with 4 ml of sterile bovine serum (total vol. of 44 ml per tube). Cells from 5 tubes were collected by centrifugation, washed twice by centrifugation in 40 ml of sterile PBS, and suspended in PBS at a cell density of approximately 1 x 10⁸ cells per ml as determined by darkfield microscopy.

A rabbit testicular antigen extract for RIA was prepared from the testicles of normal, uninfected, VDRL non-reactive rabbits for the detection of monoclonal antibodies that cross-reacted with rabbit antigens or were directed against contaminating rabbit host antigens present in T. pallidum suspensions (used for the sensitization of mice). Testicles were minced and extracted in serum-saline medium as described for T. pallidum, but centrifuged only once for 7 min at 250 x g, yielding a turbid supernatant. Ten microliters of this rabbit antigen preparation were used per microliter plate well in the RIA. Protein assays indicated that 10 microliters of this preparation contained about 120 μg of total rabbit protein and that 50% of this total protein was due to the normal rabbit serum in the serum-saline extraction medium. Preparations stored for up to 6 months at -20°C appeared to react equally as well as freshly-prepared extracts in the RIA.

Sonicated suspensions of T. pallidum or T. phagedenis biotype Reiter were also used as RIA antigen to detect antibodies directed against either "masked", subsurface or cytoplasmic treponemal antigens. Treponemal suspensions of approximately 1 x 10⁸ cells per ml in PBS were sonicated for a total of 3 min (6 min at 50% pulse) (on ice) using a stepped microtip and a Branson Sonifier Model W350 set for an output of 4 (about 40W). Ten μl of these sonicate suspensions were used per microliter plate well in the RIA. Unused suspension were stored at -20°C.

B. Plasmacytoma Cell Line

The mouse plasmacytoma cell line SP2/0-Ag14 (SP2/0), which is a hybrid cell line derived from SP2/HGLK formed from the hybridization between a BALB/c mouse spleen cell and the myeloma cell line X63/Ag8 described by Schulman et al, Nature 276:269-270 (1978) was the cell line used in this study. SP2/0 synthesizes no Ig heavy or light chains, is resistant to 20 μg of 8-azaguanine per ml, and is killed in hypoxanthine-aminopterin-thymidine (HAT) medium. SP2/0 cells are maintained in vitro in Dulbecco's Modified Eagle's Medium with high glucose (DMEM) (Grand Island Biological Company, Grand Island, New York) supplemented with 15% (vol/vol) fetal calf serum (Hy-Clone FCS; Sterile Systems, Logan, Utah), 2 mM glutamine, and 50 units of penicillin/ streptomycin (GIBCO) per ml.

4

### C. Immunization Schedule for Hybridoma Production

Adult female BALB/c mice (6-8 weeks old) were purchased from Jackson Laboratories (Bar Harbor, Maine) and used as a source of splenic lymphocytes producing antibodies against T. pallidum antigens. Mice were initially immunized with freshly-prepared T. pallidum cells in PBS ($2 \times 10^8$ cells/0.15ml PBS) in complete Freunds adjuvant (I:I) intraperitoneally (IP). Three additional injections $2 \times 10^7$ freshly isolated organisms (0.3ml PBS; IP) were given on days 2I, 42, and 63, and a final injection of $1.2 \times 10^7$ organisms in 0.4ml PBS (0.2ml IP and 0.2ml intravenously) on day 84. Three days following the last injection, spleens were removed aseptically from two mice and the spleen cells were prepared for hybridoma cell fusion by gently teasing them with forceps to prepare a single cell suspension in DMEM. The cells were then washed three times with DMEM by centrifugation at 270 x g for I0 min at 8°C.

### D. Construction of Hybridomas

Hybridomas were produced by fusing spleen cells from the immunized mice with murine SP2/0-Ag14 hybridoma cells (SP2/0 hereinafter) using a modification of the basic procedure of Kennett et al, Lymphocyte Hybridomas - Current Topics in Microbiology and Immunology, 8I, pp. 77-9I (I978) Springer-Verlag, New York. Suitable cell lines were obtained from Roger Kennett, University of Pennsylvania Medical School as originally set forth by Schulman et al, supra.

Plasmacytoma cells were harvested from culture in the logarithmic phase of growth, pelleted by centrifugation at 270 x g for I0 min, and washed three times in DMEM. Approximately I x I0$^7$ SP2/0 cells and the washed spleen cells were mixed together at a ratio of 7 viable spleen cells per viable SP2/0 cell and pelleted by centrifugation. After the supernatant was removed, 0.2 ml of warm (37°C) polyethylene glycol (35% wt/vol in DMEM; PEG 1000; J.T. Baker Chemical Company, Phillipsburg, N.J.) was added to the pellet to cause cell fusion.

The cells were then pelleted, washed in DMEM, and resuspended in 25-60 ml of DME-Hy medium (see Kennett et al, supra at p. 78) at approximately $4 \times 10^6$ cells/ml and plated under the conditions of limiting dilution in 50 $\mu$l aliquots in 96 well microliter plates (Costar Plastics, Vineland, N.J.). This fusion procedure differs from the method of Kennett et al, supra in that the SP2/0-spleen cell mixture, PEG, and DMEM are all kept at 37°C for the fusion processes. The cell pellet is loosened with a glass rod to ensure gentle mixing of the cells and the cells remain in PEG for 3 min at 37°C and for approximately 5 min. at 8°C (during centrifugation).

A two-fold concentration of HAT selection components in media, as specified in Littlefield et al, Science I45: 709-7I0 (I964, was added to each well (50 I) on day 2, followed by a I00 $\mu$l of HAT-MEDIUM on day 4. Hybrids could be observed after 6-I0 days. Wells which contained growing cells were subdivided into another 96 well plate, grown, and transferred into 24 well tissue culture plates (Costar Plastics) for maintenance. Culture supernatants were harvested for use in the screening assays for anti-T. pallidum reactivity and determination of monoclonal isotype.

### E. Characterization of Monoclonal Antibody

RIA Screening Assays for Hybridomas Secreting Monoclonal Antibodies Against T. pallidum.

To each well of a Cooke 96 well microliter plate (Dynatech Laboratories, Alexandria, Va.) was added I0 $\mu$l of a suspension or sonicate of T. pallidum or T. phagedenis biotype Reiter containing the equivalent of I x I0$^8$ cells per ml in PBS or the rabbit testicular extract described earlier. Following incubation at 4°C overnight to dryness, the wells were washed 3 times each with 0.2 ml portions of PBS containing 0.02% sodium azide to remove non-adhering antigen. Due to the strong affinity of treponemal and rabbit testicular tissue antigens for polyvinyl-chloride and other plastics, it was unnecessary to employ fixatives to promote binding of these antigens to the microliter plate wells. If desired, however, 20 $\mu$l of I0% ethanol in each well followed by evaporation to dryness at 37°C can be utilized for fixation. Wells were then precoated with 0.2 ml of PBS + I% (wt/vol) bovine serum albumin at 4°C overnight, and washed 3 times with 0.2 ml of PBS + azide. Plates were used the same day or one day following preparation.

For the RIA test, 0.05 ml of a 4 day old hybridoma clone supernatant was added to each well containing a respective antigen preparation. After 3 hr at 23°C, the wells were washed 3 times with 0.2 ml PBS + azide, followed by the addition of $2.6 \times 10^5$ CPM of a mixture of affinity purified [125] I-labeled rabbit anti-mouse IgG (specific activity of $2.0 \times 10^6$ CPM per $\mu$g) and IgM (specific activity of $3.7 \times 10^6$ CPM per $\mu$g) in 0.I ml of PBS + azide + 2% (vol/vol) horse serum per well. Binding of this probe was carried out overnight

5

at 4°C. Wells were then washed extensively with PBS and tap water. Following drying of the plates and cutting of the individual wells, counting of the wells was performed in a Nuclear Chicago model ll85 gamma counter for 0.4 minute.

Antisera used as positive controls in the RIA included l) mouse anti-T. pallidum serum collected from mice used as a source of splenic lymphocytes in cell fusions, and 2) mouse anti-rabbit testicular extract serum produced by immunizing BALB/c mice with rabbit testicular extract preparations. The mouse anti-rabbit testicular extract serum was generated by immunizing mice intraperitoneally on day l (0.25 ml testicular extract + 0.25 ml complete Freund's adjuvant) and 0.3 ml of extract on days 30 and 5l, followed by collection of the serum on day 72.

A total of 39 hybridoma cell lines were identified by RIA as secretors of monoclonal antibodies that reacted with T. pallidum antigens. Reactivity in the RIA was considered positive if $^{125}$I counts per 0.4 min were above 600 (background counts generally were in the order of 200-300). On this basis, the monoclonal antibodies arising from anti-T. pallidum hybridomas could be grouped into 3 major categories. One group of monoclonal antibodies was directed specifically against T. pallidum antigens; among these were monoclonal antibodies also capable of immobilizing virulent T. pallidum organisms in the Treponema Pallidum Immobilization test. Another group of monoclonal antibodies reacted with both T. pallidum and T. phagedenis biotype Reiter antigens and therefore was apparently directed against treponemal group antigens. A third group of monoclonal antibodies was capable of binding both treponemal and rabbit host testicular tissue antigens and thus was highly cross reactive with all antigens tested in the RIA.

Table l presents an example of the RIA protocol used to screen anti-T. pallidum hybrids. The data shown are from one of several typical RIA tests performed, and represent results characteristic of the majority of clones producing monoclonal antibodies specifically against T. pallidum. Monoclonal antibodies from such clones as those described in Table l did not react well with intact treponemes. However, they appeared to bind preferentially to sonicated antigens of T. pallidum, suggesting that they may be directed against either intracellular or "masked" antigens. Their specificity for T. pallidum determinants was indicated by their failure to cross react with T. phagedenis biotype Reiter antigen preparations and with rabbit testicular tissue antigens. These monoclonal antibodies were of mouse classes IgG and IgM, and were not reactive against T. pallidum in the TPI test. The polyclonal antisera preparations (mouse anti-T. pallidum and mouse anti-rabbit testicular tissue) used as positive controls in the RIA were strongly positive against all antigens tested. This was expected because the mouse anti-T. pallidum antibodies were derived from mice sensitized with T. pallidum suspensions containing both treponemal group antigens (that cross react with antigens of T. phagedenis biotype Reiter) as well as contaminating rabbit host antigens. Similarly, mouse anti-rabbit testicular antibodies also reacted with treponemal antigens due to the presence of shared antigens common to both rabbit host tissues and treponemes.

Monoclonal Antibody Isotype Assays.

Mouse antibody isotypes were identified by solid phase RIA. Cooke microliter plates were coated with goat anti-mouse immunoglobulin. Culture supernatants were then added and incubated for 3 hr at 37°C. Iodinated, affinity purified rabbit anti-mouse heavy chain specific reagents were added to identify the isotype of antibody bound to the plate.

Table 1. Radioimmunoassay ($^{125}I$ counts/0.4 min) on five representative hybridoma cell lines producing monoclonal antibodies against T. pallidum

| Clone | ANTIGENS | | | | | | Antibody Isotype |
|---|---|---|---|---|---|---|---|
| | T.pal[1] | T.pal-aged[2] | T.pal-son[3] | Reiter[4] | Reiter-son[5] | Rabbit[6] | |
| 9B12 | 175 | 429 | 2,594 | 173 | 200 | 118 | IgG1 |
| 6F6 | 280 | 243 | 2,455 | 206 | 219 | 233 | IgM |
| 13D4 | 187 | 274 | 2,220 | 207 | 195 | 200 | IgG1 |
| 12D10 | 270 | 215 | 2,013 | 195 | 138 | 153 | IgG2a |
| 7E3 | 226 | 189 | 1,742 | 207 | 192 | 195 | IgG3 |
| Control Antisera: | | | | | | | |
| PBS | 229 | 220 | 298 | 252 | 175 | 214 | |
| DMEM Medium | 201 | 194 | 167 | 189 | 225 | 183 | |
| Mouse anti-T. pal.[7] | 20,049 | 20,691 | 21,274 | 18,433 | 12,399 | 11,424 | |
| Mouse anti-rabbit[8] | 18,314 | 17,973 | 19,872 | 15,826 | 13,646 | 18,799 | |

1. Intact freshly isolated T. pallidum
2. Intact, "aged" T. pallidum
3. Sonicate of T. pallidum
4. Intact T. phagedenis biotype Rieter
5. Sonicate of T. phagedenis biotype Rieter
6. Rabbit testicular extract
7. 1:10 dilutions of mouse anti-T. pallidum serum from sensitized mice
8. 1:10 dilution of mouse anti-rabbit testicular tissue extract serum.

Four hybridoma cell lines secreting antibodies specifically against T. pallidum were also isolated that appeared to produce monoclonal antibodies with greater affinity for possible surface determinants of T. pallidum (see Table 2). Monoclonal antibodies from these clones possessed relatively high reactivity with both intact "aged" treponemes and sonicates of T. pallidum, but not with freshly isolated treponemes, with one possible exception, clone 7D7. Little or no cross reactivity with other treponemal or rabbit antigens was observed for these monoclonal antibodies. Despite their apparent increased specificity for possible surface components of T. pallidum, these monoclonal antibodies were also nonreactive in the TPI test.

Table 3 shows RIA data demonstrating anti-T. pallidum hybridoma cell lines producing monoclonal antibodies capable of reacting against T. pallidum in the TPI test. These clones secreted antibodies of the

7

mouse isotypes which can fix complement. The antibodies did not react very well, however, with either freshly isolated intact or "aged" intact treponemes. This observation was surprising in view of the reactive TPI test data.

Table 2. Radioimmunoassay Results on anti-T. pallidum hybridomas producing monoclonal antibodies against "aged", intact treponemes.

| Clone | ANTIGENS[1] | | | | | | Antibody Isotype |
| | T.pal | T.pal-aged | T.pal-son | Reiter | Reiter-son | Rabbit | |
|---|---|---|---|---|---|---|---|
| 11E3 | - | + | + | - | - | - | IgG2a |
| 8G2 | - | + | + | - | - | - | IgG1 |
| 5C9 | - | + | + | - | - | - | IgM |
| 7D7 | + | + | + | - | - | - | IgM |

[1]Abbreviations and controls as in Table 1.

8

Table 3. Radioimmunoassay Results on anti-T. pallidum hybridomas secreting monoclonal antibodies antibodies reactive in the TPI test.

| Clone | ANTIGENS[1] | | | | | | Antibody Isotype |
|-------|-------|------------|-----------|--------|------------|--------|------------------|
| | T.pal | T.pal-aged | T.pal-son | Reiter | Reiter-son | Rabbit | |
| 3G5 | + | − | + | − | − | − | IgM |
| 13C6 | − | +,− | + | − | − | − | IgG2a |
| 5A7 | − | − | + | − | − | − | IgM |
| 13G10 | − | − | + | − | − | − | IgG2a |
| 4117 | − | − | + | − | − | − | IgG2b |
| 13C8 | − | − | + | − | − | − | IgG2b |

[1]Abbreviations and controls as in Table 1.

Because of the multitude of antigenic similarities known to exist between members of the genus Treponema it was not unexpected to generate hybridoma cell lines producing monoclonal antibodies that cross reacted with both T. pallidum and T. phagedenis biotype Reiter antigenic determinants. However, only 3 of 39 anti-T. pallidum hybridomas isolated possessed this characteristic (Table 4).

A class of hybridoma cell lines was also isolated that produced monoclonal antibodies found to cross react with all treponemal and rabbit testicular antigens employed in the RIA (Table 5). Because of the limiting dilution procedure employed and the fact that only one antibody isotype was observed for each cell line (except one clone l2H4), it is unlikely that these hybridoma cell lines represent more than one hybridoma clone growing together in culture. It may be of interest to note that all of these highly cross reactive antibodies were of the IgM class of antibody.

Table 4. Radioimmunoassay (Results) on anti-T. pallidum hybridomas secreting monoclonal antibodies that cross react with T. phagedenis biotype Reiter.

| Clone | ANTIGENS[1] | | | | | | Antibody Isotype |
|---|---|---|---|---|---|---|---|
| | T.pal | T.pal-aged | T.pal-son | Reiter | Reiter-son | Rabbit | |
| 13A7 | - | - | + | + | + | - | IgM |
| 1B11 | - | - | + | + | - | - | IgM |
| 12G11 | - | - | + | + | + | - | IgG2b |

[1] Abbreviations and controls as in Table 1.

Table 5. Radioimmunoassay Results on hybridomas secreting monoclonal antibodies that bind T. pallidum, T. phagedenis biotype Reiter, and rabbit testicular antigens.

| Clone | ANTIGENS[1] | | | | | | | Antibody Isotype |
|---|---|---|---|---|---|---|---|---|
| | T.pal | T.pal-aged | T.pal-son | | Reiter | Reiter-son | Rabbit | |
| 1C11 | + | + | + | | + | + | + | IgM |
| 12F4 | + | + | + | | + | + | + | IgM |
| 1870-5A5 | + | + | + | | + | + | + | IgM |
| 6A9 | + | + | + | | + | + | + | IgM |
| 12E3 | - | + | + | | +,- | + | + | IgM |

[1]Abbreviations and controls as in Table 1.

Monoclonal Antibodies in the Treponema pallidum Immobilization (TPI) Test.

Mouse anti-T. pallidum serum, mouse anti-rabbit testicular extract serum, and monoclonal antibodies secreted anti-T. pallidum hybridomas were tested for their ability to immobilize virulent T. pallidum - (Nichols) in the Treponema pallidum immobilization (TPI) test. The TPI assay was carried out, with minor modifications, as described in the Manual of Tests for Syphilis (Center for Disease Control, I964, Dept. of Health, Education and Welfare, Public Health Service, N.C.D.C., Atlanta, Georgia). Penicillinase was incorporated in the test procedure due to the presence of penicillin in the hybridoma clone supernatants. Because T. pallidum was not sensitive to streptomycin at the concentrations employed in the medium, removal of streptomycin from the hybridoma clone supernatants was unnecessary.

The following hybridomas exhibited both T. Pallidum specificity and TPI-reactivity:

| CLONE | ANTIBODY ISOTYPE |
|-------|------------------|
| 3G5 | IgM |
| 4H7 | IgG2b |
| 5A7 | IgM |
| I3C6 | IgG2a |
| I3C8 | IgG2b |
| I3GI0 | IgG2a |

## Microhemagglutination (MHA-TP) Test for Treponema Pallidum Antibodies

The MHA-TP test (Sera-Tek Treponemal Antibody Test, Ames Division, Miles Laboratories, Inc.) was performed as described by the manufacturers. Briefly, 3 l of each four-day old hybridoma clone supernatant (DMEM medium) containing anti-T. pallidum monoclonal antibodies were mixed with 57 $\mu$l of absorbing diluent (I:20 dilution) and allowed to incubate at room temperature for 30 minutes. For each monoclonal antibody preparation, 25 pl aliquots of the absorbed monoclonal antibody mixture were then added in duplicate wells of a 96 well microliter plate. For each clone sample, 75 $\mu$l of sensitized sheep erythrocytes were added to one set of wells and 75 $\mu$l of unsensitized sheep erythrocytes were added to the other duplicate wells as a negative control.

Reactive clones were 8G2 and IIE3; all other hybridoma clone supernatants tested were non-reactive.

It appeared that a greater degree of anti-T. pallidum specificity was exhibited by IgG secreting clones as opposed to those clones secreting IgM. Table 6 summarizes the isotypes of all murine anti-T. pallidum monoclonal antibodies, their specificities, and their frequency of isolation in this study. A majority (24 of 3I clones) of the hybridomas isolated that reacted only against T. pallidum antigens were of IgG subclasses, compared to 7 IgM clones with similar properties. Of 8 total clones that cross reacted with either T. phagedenis biotype Reiter or rabbit testicular antigens, 7 of these were IgM producers.

TABLE 6

| Summary of monoclonal antibody characteristics from 39 anti-T. pallidum hybridomas. | | | | |
|---|---|---|---|---|
| Mouse Antibody Isotype | Antibody Specificities[1] | | | |
| | T.pal | T.pal + Reiter | T.pal + Reiter + Rabbit | Total |
| IgM | 7[2] | 2 | 5 | I4 |
| IgG3 | 2 | 0 | 0 | 2 |
| IgGI | II[4] | 0 | 0 | II |
| IgG2b | 3[2] | 1 | 0 | 4 |
| IgG2a | 7[2],[4] | 0 | 0 | 7 |
| IgGI,IgG2b[3] | I | 0 | 0 | I |
| Total | 3I | 3 | 5 | 39 |

[1]Abbreviations as in Table 1.
[2]Two of these clones were reactive in the TPI test.
[3]Clone 1939-12H4 (double producer).
[4]One of these clones was reactive in the MHA-TP test but unreactive in the TPI test.

A deposit of hybrid cell lines identified herein as 3G5 (TPI-reactive) and 8G2 (MHA-TP-reactive) are on deposit with the American Type Culture Collection and assigned the ATCC numbers HB8I33 and HB8I34, respectively.

F. Utility

The hybridoma cell lines and the monoclonal antibodies produced therefrom described herein are useful in the purification and characterization of the specific antigenic and immunogenic components presented by Treponema pallidum. Moreover, the monoclonal antibodies produced from a given hybridoma line are homogeneous in antigenic recognition and thereby are useful for subsequent affinity chromatography-based purification of Treponema antigens.

The anti-T. pallidum monoclonal antibodies can potentially be used in one or more of many ways for the development of a new diagnostic test. Various approaches can be utilized including both direct and indirect immunoassays. Variations on the general immunoassay theme include radio-immunoassay (direct or indirect), fluorescent antibody techniques (direct or indirect), enzyme-linked immunosorbent assays (ELISA's), inhibition of hemolysis assays, inhibition of agglutination tests, agglutination reactions (antibody-ligand mediated), and/or complement consumption tests. The use of one or more anti-T. pallidum monoclonal antibodies in such systems would potentially constitute an important new and useful test for the diagnosis of early primary syphilis, by virtue of the fact that the monoclonal antibodies are specific for T. pallidum and can be employed in very sensitive types of immunoassays. Such sensitive assays may be useful in the diagnosis of congenital syphilis or neurosyphilis through the detection of T. pallidum cells or antigens in congenital syphilitic lesions or in the cerebrospinal fluid of neurosyphilitics.

Moreover, the monoclonal antibodies potentially provide new affinity purification reagents that can be used to isolate and purify T. pallidum cells from rabbit tissue, which is currently performed by very expensive and inefficient methods. Through the use of monoclonal antibody affinity column chromatography, affinity purified T. pallidum cells can be prepared and employed as diagnostic antigen in currently existing serodiagnostic tests for syphilis. Such affinity purified treponemal cells may also increase the sensitivity of the pre-existing tests since this material will be much "cleaner" than conventionally prepared T. pallidum cells (i.e., free of contaminating rabbit tissue debris). Also, in lieu of whole T. pallidum cells as an antigen in serodiagnostic tests, the monoclonal antibodies may possess the ability to affinity purify newly-defined antigen components of T. pallidum cells that can be used in place of whole treponemal cells as test antigen. Consequently, the monoclonal antibodies have the ultimate potential of both identifying new antigenic components of the organism as well as the ability to purify them out from fractionated T. pallidum cells.

Ultimately, the affinity purified T. pallidum antigen components may be used to develop a vaccine for syphilis. Although only targeted segments of the U. S. population would most likely be candidates for such a vaccine (i.e., gay populations, migrant workers, inner city dwellers, etc.), there are other important treponemal diseases against which such a vaccine might be effective. Briefly, the treponemes that cause yaws, pinta, and bejel are treponemes that are morphologically and serologically indistinguishable from T. pallidum. Because of the immunological similarities of the yaws, pinta, and bejel organisms to T. pallidum, a vaccine developed against syphilis may be just as effective against these endemic treponemal diseases. Thus, the ability to prepare affinity purified antigens (immunogens) of T. pallidum that are immunogenic may have a very broad application to treponemal disease vaccines in general.

**Claims**

1. Monoclonal antibody produced by a continuous hybrid cell line, which monoclonal antibody is characterised in that:
   (i) it is capable of reacting with sonicated T. pallidum;
   (ii) it reacts with that antigenic determinant recognised by monoclonal antibodies produced by hybridoma deposit ATCC HB 8133 or HB 8134.

2. Monoclonal antibody according to claim 1, produced by a continuous hybrid cell line together with a culture medium suitable for supporting growth of the cell line.

3. Monoclonal antibody according to claim 1 or 2, wherein the hybrid cell line is a cell hybrid of:
   a) a differentiated lymphoid cell, preferably an immune spleen cell or lymph node cell, capable of producing the antibody; and
   b) a myeloma cell, preferably a hybridoma cell or plasmacytoma cell.

4. Monoclonal antibody according to claim 1 or 2, wherein the continuous hybrid cell line is a cell hybrid of a BALB/c mouse immune spleen cell capable of producing the antibody, fused to a SP2/0 hybridoma cell.

5. Monoclonal antibody according to claim 1, wherein the hybrid cell line consists essentially of hybridoma clone ATCC deposit HB8133 or HB8134.

6. Monoclonal antibody according to any preceding claim, wherein the monoclonal antibody is capable of fixing complement and immobilising virulent T. pallidum organisms.

7. Monoclonal antibody according to any preceding claim, wherein the monoclonal antibodies are capable of cross reacting among several Treponema species organisms.

8. A process for producing monoclonal antibody as defined in any preceding claim, comprising fusing a differentiated lymphoid cell capable of producing the antibody, and a myeloma cell, to provide a fused cell hybrid; isolating the hybrid; culturing the hybrid; and collecting the antibodies produced therefrom.

9. An assay method for the diagnosis of syphilis, comprising the use of a monoclonal antibody according to any of claims 1 to 7.

10. An assay method according to claim 9, wherein the monoclonal antibody is tagged with a radioisotope, a fluorescent label or an enzyme.

11. Use of a monoclonal antibody according to any of claims 1 to 7 in the purification of Treponema, especially T. pallidum, antigens.

12. Use of a monoclonal antibody according to any of claims 1 to 7 to purify Treponema, especially T. pallidum, antigens for use in the production of a vaccine therefrom.

13. Use of a monoclonal antibody according to any of claims 1 to 7 to purify Treponema, especially T. pallidum, cells for use in serodiagnostic tests.

14. A composition characterised in that it consists essentially of a continuous hybrid cell line which produces a monoclonal antibody as defined in any of claims 1 to 7.

15. A composition characterised in that it consists essentially of a monoclonal antibody according to any of claims 1 to 7.

**Patentansprüche**

1. Monoklonaler Antikörper, der durch eine kontinuierliche Hybridzellinie erzeugt wird, dadurch gekennzeichnet, daß er
   (i) mit beschalltem T. pallidum reagieren kann;
   (ii) mit derjenigen antigenen Determinante reagiert, die durch von der Hybridom-Hinterlegung ATCC HB 8133 oder HB 8134 erzeugten monoklonalen Antikörper erkannt wird.

2. Monoklonaler Antikörper nach Anspruch 1, der durch eine kontinuierliche Hybridzellinie zusammen mit einem Kulturmedium erzeugt wird, das für die Unterstützung des Wachstums der Zellinie geeignet ist.

3. Monoklonaler Antikörper nach Anspruch 1 oder 2, bei welchem die Hybridzellinie ein Zellhybrid ist von:
   (a) einer differenzierten Lymphoidzelle, vorzugsweise einer immunen Milzzelle oder Lymphknotenzelle, die zur Erzeugung des Antikörpers in der Lage ist, und
   (b) einer Myelomzelle, vorzugsweise einer Hybridomzelle oder Plasmozytomzelle.

4. Monoklonaler Antikörper nach Anspruch 1 oder 2, bei welchem die kontinuierliche Hybridzellinie ein Zellhybrid einer BALB/c-Maus-Immun-Milzzelle ist, die zur Erzeugung des Antikörpers in der Lage ist und mit einer SP2/0-Hybridomzelle verschmolzen ist.

5. Monoklonaler Antikörper nach Anspruch 1, bei welchem die Hybridzellinie im wesentlichen aus der Hybridomklon-Hinterlegung ATCC HB 8133 oder HB 8134 besteht.

6.  Monoklonaler Antikörper nach einem der vorstehenden Ansprüche, bei welchem der monoklonale Antikörper zur Komplementbindung und Immobilisierung virulenter T. pallidum-Organismen in der Lage ist.

7.  Monoklonaler Antikörper nach einem der vorstehenden Ansprüche, bei welchem die monoklonalen Antikörper zur Kreuzreaktion mit mehreren Organismen der Treponema-Spezies in der Lage sind.

8.  Verfahren zur Erzeugung monoklonaler Antikörper, wie in den vorstehenden Ansprüchen festgelegt, umfassend das Verschmelzen einer differenzierten Lymphoidzelle, die zur Erzeugung des Antikörpers in der Lage ist, und einer Myelomzelle, um einen verschmolzenen Zellhybrid zu schaffen; das Isolieren des Hybrids, das Inkulturnehmen des Hybrids und das Sammeln der daraus erzeugten Antikörper.

9.  Analysenmethode zur Diagnose der Syphilis, umfassend die Verwendung eines monoklonalen Antikörpers nach einem der vorstehenden Ansprüche 1 bis 7.

10. Analysenmethode nach Anspruch 9, bei welcher der monoklonale Antikörper mit einem Radioisotop, einer Fluoreszenzmarkierung oder einem Enzym markiert ist.

11. Verwendung eines monoklonalen Antikörpers nach einem der Ansprüche 1 bis 7 bei der Reinigung von Antigenen der Trenonema, insbesondere T. pallidum.

12. Verwendung eines monoklonalen Antikörpers nach einem der Ansprüche 1 bis 7 zur Reinigung von Antigenen der Treponema, insbesondere T. pallidum, zum Einsatz bei der Erzeugung eines Impfstoffes daraus.

13. Verwendung eines monoklonalen Antikörpers nach einem der Ansprüche 1 bis 7 zur Reinigung von Zellen der Treponema, insbesondere T. pallidum, bei Verwendung in serodiagnostischen Tests.

14. Zusammensetzung, dadurch gekennzeichnet, daß sie im wesentlichen aus einer kontinuierlichen Hybridzellinie besteht, die einen monoklonalen Antikörper erzeugt, wie er in einem der Ansprüche 1 bis 7 festgelegt wurde.

15. Zusammensetzung, dadurch gekennzeichnet, daß sie im wesentlichen aus einem monoklonalen Antikörper gemäß einem der Ansprüche 1 bis 7 besteht.

**Revendications**

1.  Anticorps monoclonal produit par une lignée cellulaire continue, lequel anticorps monoclonal est caractérisé en ce que:
    (i) il est capable de réagir avec le T-pallidum qui a été soumis à un traitement aux ultra-sons;
    (ii) il réagit avec le déterminant antigénique qui est reconnu par les anticorps monoclonaux produits par le dépôt d'hybridome ATCC HB 8133 ou HB 8134.

2.  Anticorps monoclonal selon la revendication 1, produit par une lignée cellulaire hybride continue avec un milieu de culture convenable pour supporter le développement de la lignée cellulaire.

3.  Anticorps monoclonal selon la revendication 1 ou 2, dans lequel la lignée cellulaire hybride est un hybride cellulaire de
    a) une cellule lymphoïde différenciée, de préférence une cellule immunitaire de la rate ou une cellule de ganglion lymphatique capable de produire l'anticorps et
    b) une cellule de myélome, de préférence une cellule d'hybridome ou une cellule de plasmacytome.

4.  Anticorps monoclonal suivant la revendication 1 ou 2, dans lequel la lignée cellulaire hybride continue est un hybride cellulaire d'une cellule immunitaire de la rate de souris BALB/C capable de produire l'anticorps fusionnée avec une cellule d'hybridome SP2/0.

5.  Anticorps monoclonal suivant la revendication 1, dans lequel la lignée cellulaire hybride consiste essentiellement dans le clone d'hybridome déposé à l'ATCC sous le numéro HB 8133 ou HB 8134.

15

**6.** Anticorps monoclonal suivant l'une quelconque des revendications précédentes, dans lequel l'anticorps monoclonal est capable de fixer le complément et d'immobiliser les organismes virulents de T-pallidum.

**7.** Anticorps monoclonal suivant l'une quelconque des revendications précédentes, dans lequel les anticorps monoclonaux sont capables de donner une réaction croisée avec plusieurs organismes de l'espèce Treponema.

**8.** Un procédé de production d'un anticorps monoclonal tel que défini dans l'une quelconque des revendications précédentes, lequel procédé comprend la fusion d'une cellule lymphoïde différenciée capable de produire l'anticorps et d'une cellule de myélome pour donner un hybride de cellules fusionnées, l'isolement de l'hybride, la culture de l'hybride et la récolte des anticorps qui en sont produits.

**9.** Une méthode de test pour le diagnostic de la syphilis, comprenant l'usage d'un anticorps monoclonal suivant l'une quelconque des revendications 1 à 7.

**10.** Une méthode de test suivant la revendication 9, dans laquelle l'anticorps monoclonal est marqué avec un radioisotope, une marque fluorescente ou une enzyme.

**11.** Usage d'un anticorps monoclonal suivant l'une quelconque des revendications 1 à 7 dans la purification d'antigènes de Treponema, spécialement de T-pallidum.

**12.** Usage d'un anticorps monoclonal suivant l'une quelconque des revendications 1 à 7 pour purifier des antigènes de Treponema, spécialement de T-pallidum pour un usage dans la production d'un vaccin au départ desdits antigènes.

**13.** Usage d'un anticorps monoclonal suivant l'une quelconque des revendications 1 à 7 pour purifier des cellules de Treponema, spécialement de T-pallidum pour un usage dans des tests de sérodiagnostic.

**14.** Composition caractérisée en ce qu'elle consiste essentiellement en une lignée cellulaire hybride continue qui produit un anticorps monoclonal tel que défini dans l'une quelconque des revendications 1 à 7.

**15.** Composition caractérisée en ce qu'elle consiste essentiellement en un anticorps monoclonal selon l'une quelconque des revendications 1 à 7.